(19) Europäisches Patentamt
European Patent Office
Office européen des brevets

(11) **EP 4 564 360 B1**

(12) **EUROPEAN PATENT SPECIFICATION**

(45) Date of publication and mention
of the grant of the patent:
**28.01.2026 Bulletin 2026/05**

(51) International Patent Classification (IPC):
**A61M 5/172** (2006.01)   **G16H 20/40** (2018.01)
**G16H 50/50** (2018.01)

(21) Application number: **23213402.3**

(22) Date of filing: **30.11.2023**

(52) Cooperative Patent Classification (CPC):
**G16H 20/17; A61M 5/1723; G16H 20/40;
G16H 50/50;** A61M 2230/20

(54) **MANAGEMENT OF FLUID ADMINISTRATION**

VERWALTUNG DER FLÜSSIGKEITSVERABREICHUNG

GESTION DE L'ADMINISTRATION DE FLUIDE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**04.06.2025 Bulletin 2025/23**

(73) Proprietor: **TOB1 Consulting Ltd
London EN1 2PH (GB)**

(72) Inventor: **O'BRIEN, Terence
London, EN1 2PH (GB)**

(74) Representative: **Barker Brettell LLP
100 Hagley Road
Edgbaston
Birmingham B16 8QQ (GB)**

(56) References cited:
**US-A1- 2010 081 942**

• HAHN ROBERT G ET AL: "Volume Kinetics for
Infusion Fluids", ANESTHESIOLOGY
(PHILADELPHIA), 1 August 2010 (2010-08-01),
Hagerstown, MD, pages 470 - 481, XP093151493,
Retrieved from the Internet <URL:https://pubs.
asahq.org/anesthesiology/article-pdf/113/2/470/
250740/0000542-201008000-00035.pdf>
[retrieved on 20240415], DOI: 10.1097/
ALN.0b013e3181dcd88f
• CHOI BYUNG-MOON: "Interpretation of volume
kinetics in terms of pharmacokinetic principles",
KOREAN JOURNAL OF ANESTHESIOLOGY, vol.
74, no. 3, 5 March 2021 (2021-03-05), KR, pages
204 - 217, XP093151486, ISSN: 2005-6419, DOI:
10.4097/kja.21085

**Description**

The invention relates generally to a system for management of administration of intravenous fluids. More particularly, but not exclusively, the invention relates to a system for management of administration of intravenous fluids following use of an anaesthetic.

Background

At least 310 million patients undergo major surgery worldwide every year and these procedures often require the use of an anesthetic. Administration of an anesthetic typically causes vasodilation of the vascular system, this increase in capacitance reduces venous return which leads to a drop in blood flow and mean arterial pressure ("MAP"). **In** order to maintain blood flow and keep organs perfused with oxygen, medical practitioners may need to administer an initial dose of intravenous fluids to increase the circulating blood volume and restore the patient's mean arterial pressure, to a pressure adequate to restore organ perfusion - usually at least 65 mmHg. The initial dose of intravenous fluid is known as resuscitating fluid, following which a maintenance infusion of fluid is administered to maintain blood volume by matching losses from the vascular system. However, administering intravenous fluids purely based on mean arterial pressure can be problematic.

Whilst the patient is under the anesthetic, fluid exits the central blood compartment, for example, through urine excretion via the kidneys, through insensible losses via the skin and respiration, and through diffusion into the tissue interstitial fluid compartment. These losses, if uncompensated for, lead to a drop in circulating blood volume that results in a lower than desired MAP or systolic arterial pressure. To maintain the patient's blood volume and arterial pressure at a certain level, as previously mentioned, a maintenance intravenous fluid is provided, for example, via a continuous intravenous drip.

Using traditional intravenous fluid delivery methods, as described above, medical practitioners may end up delivering an excess of fluid, sometimes as much as multiple litres of fluid to the patient on the day of surgery, resulting in intra operative fluid overload and marked post operative tissue edema. Judging the amount of fluid to maintain an adequate blood volume under anesthesia is currently very difficult. Fluid overload is a therefore a frequent consequence of anesthesia and is associated with postoperative complications that include increasing the risk of poor wound healing, pulmonary congestion, and pulmonary edema.

Restricting fluids to attempt to achieve a fluid balance is a potential alternative strategy to the intravenous fluid delivery methods described above, but this is also associated with complications. Restricting fluid delivery has become a key component of enhanced recovery after surgery (ERAS) pathways. The largest study of perioperative (in and around the time of surgery) fluid management to date, the REstrictive Versus LIbEral Fluid Therapy in Major Abdominal Surgery (RELIEF) trial of 3000 patients showed, that in comparison with a modestly liberal fluid volume, restrictive fluid administration led to comparable survival at 12 months, but was associated with significantly greater incidence of renal dysfunction and surgical site infections. Fluid restriction increases the risk of low blood flow and hence low blood pressure (hypotension), decreases perfusion in the kidney and other vital organs leading to organ dysfunction, kidney damage, increased infections and other complications.

US2010081942 A1 describes a method and apparatus for monitoring fluid balance status of a subject. A hemoglobin measure indicative of hemoglobin concentration in the blood of a subject and at least one physiological parameter responsive to blood volume changes in the subject are determined and concurrent behavior of the hemoglobin measure and the at least one physiological parameter is indicated to a user, thereby to give an indication of fluid balance status of the subject. The cause and/or reliability of the fluid balance status may also be indicated.

The present invention was devised with foregoing in mind.

Summary of Invention

According to a first aspect of the invention, there is provided a system for managing fluid administration to a patient following administration of an anaesthetic.

The system comprises a processor. The processor is configured to receive monitored patient haemoglobin data before and after the administration of the anaesthetic and a resuscitating fluid to the patient. Haemoglobin data may comprise haemoglobin concentration. Haemoglobin data may comprise haematocrit (i.e., the percentage volume occupied by red blood cells). Haemoglobin data may comprise the packed red cell volume (i.e., the actual volume of blood occupied by red blood cells).

The processor is configured to calculate a baseline blood volume ($_BBV$) of the patient.

The processor is configured to, after administration of the anaesthetic, calculate a target blood volume ($_TBV$). For situations in which a medical professional administers a resuscitating fluid bolus to the patient following administration of the anaesthetic, the processor may be configured to, after administration of the anaesthetic and the initial resuscitating fluid, calculate the target blood volume ($_TBV$).

The processor is configured to calculate the target blood volume ($_TBV$) by comparing the monitored patient haemoglobin data before the administration of the anaesthetic with the monitored patient haemoglobin data after the administration of the anaesthetic. The processor may be configured to calculate the target blood volume ($_TBV$) by comparing the monitored patient haemoglobin data before the administration of the anaesthetic and resuscitating fluid with the monitored patient haemoglobin data after the administration of the anaesthetic and resuscitating fluid. When no resuscitating fluid is given to the patient, the monitored patient haemoglobin data before the administration of the anaesthetic and the monitored patient haemoglobin data after the administration of the anaesthetic may be unchanged.

Calculating a target blood volume enables medical professionals to ensure that they are not providing excess amounts of fluid to the patient. This reduces the risk of under filling the circulation and reduces the risk of fluid overfilling which causes unnecessary dilution of the haemoglobin concentration and reduces the likelihood of medical conditions that arise from having excess levels of fluid in the interstitial fluid compartment.

The processor may be configured to receive user characteristics. The processor may be configured to calculate the baseline blood volume ($_BBV$) using the user characteristics. The processor may be configured to calculate the baseline blood volume ($_BBV$) using the user characteristics using Nadler's method.

Calculating the baseline blood volume using user characteristics enables a fast and simple estimate for the baseline blood volume, which in turn simplifies the calculation of the target blood volume.

The processor may be configured to calculate the target blood volume ($_TBV$) by multiplying the baseline blood volume ($_BBV$) by a dilution factor. The dilution factor may be equal to the haemoglobin concentration following administration of the resuscitating bolus divided by the haemoglobin concentration prior to administration of the anaesthetic.

When no resuscitating fluid is given to the patient and the monitored patient haemoglobin data before the administration of the anaesthetic and the monitored patient haemoglobin data after the administration of the anaesthetic is unchanged, the processor may set the target blood volume ($_TBV$) to be equal to the baseline blood volume ($_BBV$). In other words, the dilution factor may be equal to 1.

The processor is configured to receive ongoing monitored patient haemoglobin data. Ongoing patient haemoglobin data may comprise repeated or periodic haemoglobin concentration measurements. Ongoing patient haemoglobin data may be a real time stream of haemoglobin concentration measurements. The period between successive haemoglobin measurements may be multiple minutes, less than 1 minute, or less than 30 seconds, or less than 10 seconds, or less than 1 second.

The processor is configured to calculate a monitored blood volume ($_MBV$) based on the ongoing monitored patient haemoglobin data. The processor may be configured to calculate the monitored blood volume ($_MBV$) based on a most recent measured value of haemoglobin concentration. The processor may be configured to calculate the monitored blood volume ($_MBV$) based on a most recent measured value of haematocrit. The processor may be configured to calculate the monitored blood volume ($_MBV$) based on a most recent measured value of packed red cell volume.

The processor may be configured to calculate the monitored blood volume ($_MBV$) by multiplying the baseline blood volume ($_BBV$) by a dilution factor. The dilution factor may be the most recent measured value of haemoglobin concentration divided by the haemoglobin concentration measured prior to administration of the anaesthetic. The dilution factor may be the most recent measured value of haematocrit divided by the value of haematocrit measured prior to administration of the anaesthetic. The dilution factor may be the most recent measured value of packed red cell volume divided by the value of packed red cell volume measured prior to administration of the anaesthetic. The processor may be configured to calculate the monitored blood volume ($_MBV$) by multiplying the target blood volume ($_TBV$) by a dilution factor. The dilution factor may be the most recent measured value of haemoglobin concentration divided by the haemoglobin concentration measured following administration of the resuscitating fluid bolus and used to obtain the target blood volume ($_TBV$). The dilution factor may be the most recent measured value of haematocrit divided by the value of haematocrit measured following administration of the resuscitating fluid bolus and used to obtain the target blood volume ($_TBV$). The dilution factor may be the most recent measured value of packed red cell volume divided by the value of packed red cell volume measured following administration of the resuscitating fluid bolus and used to obtain the target blood volume ($_TBV$).

The processor may be configured to calculate the monitored blood volume ($_MBV$) by multiplying a previous value of the monitored blood volume ($_MBV$) by a dilution factor. The dilution factor may be the most recent measured value of haemoglobin concentration divided by the haemoglobin concentration measured to obtain the previous monitored blood volume ($_MBV$). The dilution factor may be the most recent measured value of haematocrit divided by the value of haematocrit measured to obtain the previous monitored blood volume ($_MBV$). The dilution factor may be the most recent measured value of packed red cell volume divided by the value of packed red cell volume measured to obtain the previous monitored blood volume ($_MBV$).

The processor may be configured to receive a net change in haemoglobin content, haematocrit or packed red cell volume. The processor may be configured to calculate a net change in haemoglobin content, haematocrit or packed red cell volume. The processor may be configured to calculate the net change in haemoglobin, haematocrit or packed red cell volume based on a measured, or estimated, blood loss and/or a blood transfusion. The processor may be configured to calculate the monitored blood volume ($_M$BV) based on the ongoing monitored patient haemoglobin data together with the net change in haemoglobin, haematocrit or packed red cell volume content.

Taking net change in haemoglobin, haematocrit or packed red cell volume into account when calculating the monitored blood volume ensures that haemoglobin concentration, haematocrit or packed red cell volume can still be used to determined circulating blood volume.

The processor may be configured to calculate a central compartment fluid loss rate.

Calculating a central compartment fluid loss rate enables a medical professional to provide a maintenance fluid with a flow rate configured to increase, decrease, or maintain the circulating blood volume in the central compartment (i.e., the maintenance fluid rate can be determined responsive to the fluid loss rate, to balance fluid losses, and optionally any further changes in capacitance of the central blood compartment).

The processor may be configured to update the central compartment fluid loss rate such that it is individualized to the patient.

Having an individualised central compartment fluid loss rate enables a medical professional to provide a maintenance fluid with a flow rate configured to increase, decrease, or maintain the circulating blood volume in the central compartment with greater accuracy.

The processor may be configured to calculate the central compartment fluid loss rate using a plurality of flow rate constants.

The use of multiple flow rate constants enables the medical professional to recognise where the fluid is going once it exits the central compartment.

The processor may be configured to update the flow rate constants such that they are individualized to the patient.

The use of multiple individualised flow rate constants enables the medical professional to recognise where the fluid is going once it exits the central compartment with greater accuracy.

The processor may be configured to receive measured urinary output. The processor may be configured to receive measured blood loss or blood gain. The processor may be configured to update the flow rate constants such that they are individualized to the patient based on the measured urinary output and measured blood loss or blood gain.

The flow rate constants may comprise one or more of:

(i) rate of fluid loss to the urinary system of the patient;
(ii) rate of fluid loss to the interstitial fluid compartment of the patient;
(iii) rate of fluid gain from the interstitial fluid compartment of the patient;
(iv) rate of insensible fluid losses; and
(v) rate of blood loss.

The processor is configured to calculate a predicted future blood volume ($_P$BV). The processor may be configured to calculate the predicted future blood volume ($_P$BV) using the central compartment fluid loss rate.

The processor is configured to calculate, based on the predicted future blood volume ($_P$BV) and the target blood volume ($_T$BV), one or more of:

(i) a recommended maintenance fluid rate to maintain $_M$BV at, or near to, $_T$BV; and
(ii) a recommended resuscitative fluid bolus volume and rate of administration to bring $_M$BV to, or near to, $_T$BV.

The processor may be configured to calculate a flow rate constant for accumulation of fluid in the interstitial fluid compartment.

The processor may be configured to estimate an initial interstitial fluid volume. The processor may be configured to calculate a monitored interstitial fluid volume ($_M$ISF). The processor may be configured to calculate an increase in interstitial fluid volume. The processor may be configured to calculate a monitored interstitial fluid volume ($_M$ISF) by adding the increase in interstitial fluid volume to the initial interstitial fluid volume.

The system may comprise a display. The display may be configured to show one or more of: target blood volume ($_T$BV), monitored blood volume ($_M$BV), the difference between the target blood volume ($_T$BV) and the monitored blood volume ($_M$BV), the monitored patient haemoglobin data, the oxygen delivery rate, the target oxygen delivery rate, mean arterial pressure, target mean arterial pressure, the interstitial fluid volume, and one or more recommended actions.

According to a non-claimed aspect of the invention, there is provided a system for training management of fluid administration to a patient following administration of an anaesthetic. The system comprises a processor and a display. The processor may be the processor according to the first aspect of the invention.

The system according to the second aspect may be configured to to simulate patient response to fluid resuscitation and maintenance fluid. The system may be configured to display changes in central volume and interstitial volume during the course of a simulated medical procedure. The system may be used to train medical professionals. The system may comprise a user interface with control elements for the user to define and virtually administer a resuscitation fluid and any fluid bolus and/or maintenance flow, blood products, various cardiac and vasoactive drugs. The user interface may comprise one or more control elements (e.g., buttons, sliders, dialog boxes) for input of maintenance fluid flow rates, fluid bolus injection (volume and rate), and resuscitation volume (volume and rate). The user interface may comprise any of the graphics and features described with reference to any aspect or embodiment of the invention, which may assist the user to determine an appropriate fluid management strategy.

**In** some scenarios, the system may withhold some parameters from being displayed, such as the target blood volume - in order to emphasize the value of these parameters in appropriate fluid management.

The simulation may be based on a model determined by analysis of real patient data from a surgical procedure. For example, the fluid loss terms discussed above (with reference to the model) may be determined for a real procedure, and a user may be tested using the realistic data derived from the scenario to administer fluid appropriately. A scoring metric may be determined from the simulation, which may be based on one or more of: maintenance of a suitable MAP (positive score), suitable oxygen delivery without excessive hemodilution and requirement for excessive inotrope support (positive score), excess interstitial fluid (negative score).

The simulation may also facilitate retrospective analysis of real scenarios - to illustrate whether performance in a real setting was optimal or not. Analysis of such historical information may provide an invaluable training aid.

According to another non-claimed aspect of the invention, there is method for managing fluid administration to a patient following administration of an anaesthetic.

The method may comprise:

obtaining patient haemoglobin data before and after the administration of the anaesthetic to the patient; calculating a baseline blood volume ($_B$BV) of the patient; and after administration of the anaesthetic, calculating a target blood volume ($_T$BV), wherein the target blood volume ($_T$BV) is calculated by comparing the monitored patient haemoglobin data before and after the administration of the anaesthetic.

The method may comprise:

obtaining patient haemoglobin data before and after the administration of the anaesthetic and a resuscitating fluid to the patient; calculating a baseline blood volume ($_B$BV) of the patient; and after administration of the anaesthetic and the resuscitating fluid, calculating a target blood volume ($_T$BV), wherein the target blood volume ($_T$BV) is calculated by comparing the monitored patient haemoglobin data before and after the administration of the anaesthetic and resuscitating fluid.

The method may comprise:

obtaining patient haemoglobin data before and after the administration of the anaesthetic and, if required, a resuscitating fluid, to the patient; calculating a baseline blood volume ($_B$BV) of the patient; and after administration of the anaesthetic and the resuscitating fluid, calculating a target blood volume ($_T$BV), wherein the target blood volume ($_T$BV) is calculated by comparing the monitored patient haemoglobin data before and after the administration of the anaesthetic and resuscitating fluid.

Any calculations performed by the processor of the first aspect of the invention may be recited as steps in the method of the third aspect of the invention.

Optional features of any of the above aspects may be combined with the features of any other aspect, in any combination. For example, features described in connection with the system of the first aspect may have corresponding features definable with respect to the method of the third aspect, and vice versa, and these embodiments are specifically envisaged. Features which are described in the context or separate aspects and embodiments of the invention may be used together and/or be interchangeable wherever possible. Similarly, where features are, for brevity, described in the context of a single embodiment, those features may also be provided separately or in any suitable subcombination.

Brief description of the drawings

The invention will now be described, by way of example only, with reference to the accompanying drawings, in which:

**Figure 1** shows a schematic view of a system according to the present invention;

**Figure 2** shows a schematic view of a medical professional using the system of the present invention;

**Figure 3** shows a schematic view of a model representing fluid flow within a patient; and

**Figure 4** shows an oxygen delivery screen used in accordance with an embodiment of the invention;

**Figure 5** shows a perfusion screen used in accordance with an embodiment of the invention; and

**Figure 6** shows a fluid challenger calculator used in accordance with an embodiment of the invention.

<u>Detailed description</u>

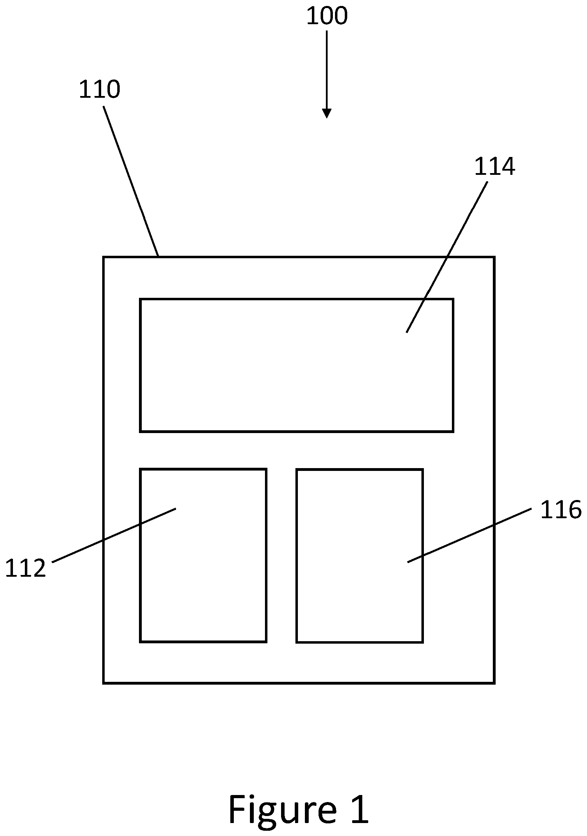

Figure 1 shows a schematic view of a system 100 for managing fluid administration to a patient following administration of an anaesthetic, according to an embodiment of the invention.

The term "anaesthetic" used herein is intended to refer to any medicine which a doctor, anaesthetist, or other medical professional may administer to a patient prior to surgery. The anaesthetic can be a general anaesthetic, a local anaesthetic, or a sedative. The term "medical professional" used herein is intended to refer to one or more person(s) monitoring a patient before, during, and/or after the use of an anaesthetic.

The system 100 comprises an apparatus 110. The apparatus 110 comprises a processor 112, a display 114, and a user interface 116.

The processor 112 can be a microprocessor. In some embodiments, the processor 112 can communicate with an external server which performs calculations and returns values to the processor. The processor may communicate with (i.e., receive data/values from) the user interface 116. The processor 112 may be integrated into a patient monitoring device.

The display 114 can be a visual display. This display 114 is used to display various parameters which are calculated by, or inputted to, the processor 112. The display 114 can display any of the values calculated by the processor 112 and/or any data received by the processor 112. The display 114 can be configured to communicate recommendations provided by the processor 112 to medical professionals. The display 114 can display any measured or calculated values discussed herein.

The user interface 116 can be a touch screen, keyboard, or any other suitable interface. In alternative embodiments, the user interface 116 can be replaced with a wired and/or wireless communications system which enables communication with an external user device (e.g., smartphone or tablet), and data can be input to the processor 112 via the external user device.

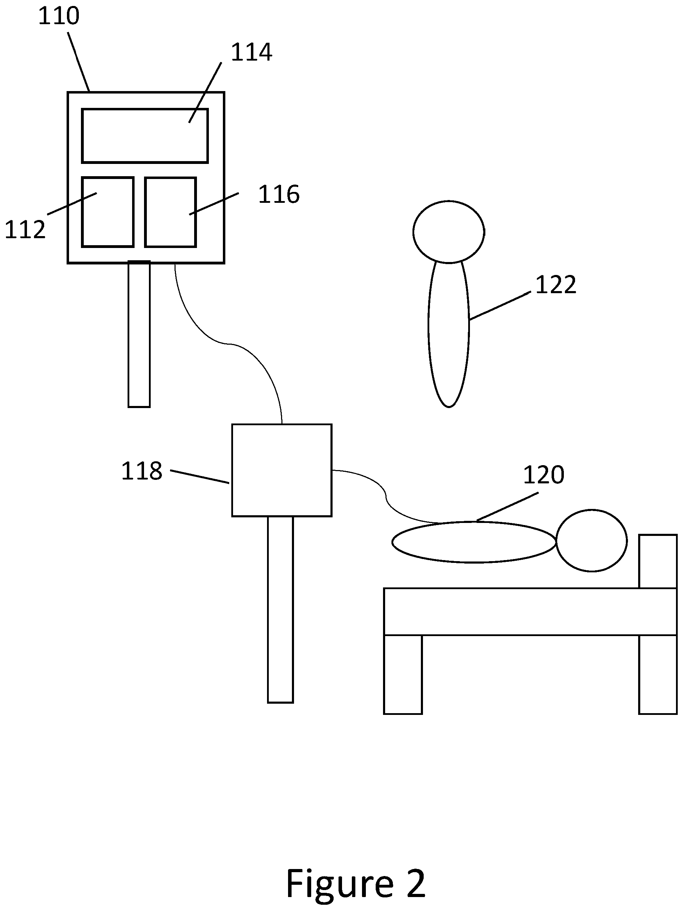

Figure 2 shows a schematic view of a patient 120 being monitored whilst under the influence of an aesthetic.

The patient 120 is connected to a patient monitoring device 118. The patient monitoring device 118 is configured to measure one or more physiological parameters of the patient 120, such as mean arterial blood pressure, heart rate, arterial oxygen saturation, cardiac output, or any other physiological parameters discussed herein. Although one patient monitoring device 118 is shown in Figure 2, a plurality of patient monitoring devices can be used instead. The patient monitoring device 118 can obtain regular measurements (e.g., every heartbeat, respiratory cycle, 2 minutes, 5 minutes, 10 minutes, etc.) or provide a real-time stream of measurements or substantially real-time stream of measurements (e.g., measurements separated by 1 second or less).

The patient monitoring device 118 is connected to the apparatus 110 such that physiological parameters measured by the patient monitoring device 118 are directly input to the processor 112 of the apparatus 110. In alternative uses, the monitoring device 118 is not connected to the apparatus 110, and a medical professional 122 inputs data obtained by the patient monitoring device 118 to the apparatus 110 via the user interface 116.

In addition to the physiological parameters measured by the patient monitoring device 118, patient data (e.g., height, weight, age, sex) is input to the processor 112 of the apparatus 110 by the medical professional 122 (or may be received electronically from a further computer system that includes patient records).

The processor 112 is configured to receive monitored patient haemoglobin data. Patient haemoglobin data comprises the concentration of haemoglobin within the blood of the patient 120. In some embodiments, the monitored patient haemoglobin data may comprise patient haematocrit or packed red cell volume.

The patient haemoglobin data can be obtained via analysis of a blood sample taken from the patient 120, for example, by the medical professional 122. In such embodiments, the medical professional 122 inputs the monitored patient haemoglobin data to the processor 112 via the user interface 116 (or the data may be transmitted to the processor 112 electronically from an input terminal which may be remote from the device 118). The medical professional 122 may regularly (i.e., periodically) input monitored patient haemoglobin data to the processor 112. In other embodiments, a haemoglobin monitoring device can be worn by the patient which transmits patient haemoglobin data to directly the apparatus 110. The haemoglobin monitoring device can provide a stream of real-time measurements of the patient's haemoglobin concentration, and this data can be input directly to the processor 112.

The processor 112 can be configured to calculate a bound oxygen delivery rate of the patient 120 based on the monitored patient haemoglobin data. The processor 112 can calculate the bound oxygen delivery rate of the patient 120 based on the monitored patient haemoglobin data together with additional physiological parameters of the patient 120, such as those obtained via the patient monitoring device 118. The skilled person will recognise that the bound oxygen delivery rate is given by the product of the cardiac output (heart rate multiplied by stroke volume) with the arterial oxygen content. Therefore, the processor 112 can use the monitored patient haemoglobin data together with monitored physiological parameters to calculate the arterial oxygen content and thus the bound oxygen delivery rate. The display 114 can display the bound oxygen delivery rate.

The bound oxygen delivery rate represents the majority of the transported oxygen within the body. In some embodiments, oxygen tension values are also provided to the processor 112 (e.g., via the patient monitoring device 118 or the medical professional 122) such that the processor 112 can determine the non-bound dissolved oxygen content in the blood and the total (i.e., the combined bound and unbound oxygen) oxygen delivery rate.

The processor 112 is configured to calculate a baseline blood volume ($_B$BV) of the patient 120. The baseline blood volume ($_B$BV) of the patient 120 is the volume of blood circulating in the patient's central blood compartment (i.e., the circulating blood volume prior to the administration of an anaesthetic or resuscitating fluid bolus). The central blood compartment ($V_C$) refers to volume occupied defined by the patient's veins/venules, arteries, capillaries, and heart chambers.

In some embodiments, the processor 112 is configured to receive patient characteristics, such as patient height, patient weight, patient age, and patient sex. In such embodiments, the processor 112 is configured to calculate the baseline blood volume ($_B$BV) using the patient characteristics. For example, the processor 112 can calculate the baseline blood volume ($_B$BV) using Nadler's formula. In other embodiments, the processor 112 calculates the baseline blood volume ($_B$BV) using alternative methods, which may or may not use the aforementioned patient characteristics. The patient characteristics can be input to the processor 112 by the medical professional 122.

Prior to surgery, a medical professional 122 administers an anaesthetic. Upon administration of the anaesthetic, the patient's vascular system undergoes vasodilation, an effect of which is that the capacitance of the central blood compartment ($V_C$) increases. The increased central blood compartment ($V_C$) capacitance leads to a drop in the stressed vascular volume and increase in the unstressed vascular volume, which has the effect of reducing the venous pressure and the blood return rate to the heart. As the heart cannot pump out more blood than it gets back from circulation, the decrease in blood return rate decreases the cardiac output, resulting in a reduction in mean arterial pressure. To provide hemodynamic stability and to ensure a sufficient level of organ perfusion, the medical professional 122 administers a resuscitating fluid bolus to increase the circulating blood volume within the central blood compartment ($V_C$). Increasing the circulating blood volume at this stage increases the patient's cardiac output and oxygen delivery rate.

The resuscitating fluid is provided as an intravenous bolus or fast flow from an infusion pumping device. The resuscitating fluid bolus increases the circulating blood volume beyond the baseline blood volume ($_B$BV) and increases the venous return and cardiac output to regain hemodynamic stability post-anaesthesia.

The volume of resuscitating fluid given is typically not individualised to the patient 120 and is determined, typically, by the medical professional 122. The resuscitating fluid bolus is typically in the range of 0 -750ml, but the volume and its rate of administration depends on various factors, including baseline blood volume ($_B$BV), mean or systolic arterial pressure drop, blood pressure target level, anaesthetic type/depth, heart contractility and heart rate.

The resuscitating fluid is administered intravenously into the central blood compartment ($V_C$). However, the body is constantly redistributing and eliminating fluid from the central blood compartment ($V_C$) (for example, blood loss, redistribution to the interstitial fluid compartment, elimination through urine output, and elimination through insensible losses), meaning over time a variable amount of the resuscitating fluid remains in the central blood compartment ($V_C$).

The fluid from the resuscitating fluid bolus which remains in the central blood compartment ($V_C$) causes a fall, through dilution, in the haemoglobin concentration of the patient's blood. The fall in haemoglobin concentration can be calculated by comparing the monitored patient haemoglobin data before and after the administration of the anaesthetic and resuscitating fluid bolus. The degree of haemoglobin dilution can be used to determine the change in the circulating blood volume within the central blood compartment (Vc).

After the resuscitating fluid bolus has been administered, and the patient's mean arterial pressure has been restored to a safe level (for example, greater than or equal to 65 mmHg), the circulating blood volume within the central compartment ($V_C$) is calculated. The calculated circulating blood volume is set as the target blood volume ($_T$BV), as it is the volume of blood within the central compartment $V_C$ which the medical professional 122 considers to be sufficient to maintain organ perfusion (i.e., the venous return, cardiac output, mean arterial pressure and oxygen delivery throughout the body is at a sufficient level). To counter-act the ongoing background fluid losses from the central compartment described above, the medical professional 122 provides a maintenance fluid to the patient 120, which is a constant supply of fluid administered intravenously to the patient 120.

The processor 112 is configured to calculate the target blood volume ($_T$BV). The processor 112 is configured to calculate the target blood volume ($_T$BV) based on the baseline blood volume ($_B$BV) and the change in concentration of haemoglobin following administration of the anaesthetic and resuscitating fluid bolus.

The target blood volume ($_T$BV) is calculated using the baseline blood volume ($_B$BV), together with the monitored patient haemoglobin data before and after the administration of the anaesthetic and resuscitating fluid. For a known initial blood volume (i.e., the baseline blood volume $_B$BV) and haemoglobin concentration, the new blood volume can be determined from the new haemoglobin concentration using the assumption that no haemoglobin is lost during the period covering the administration of the anaesthetic, resuscitating fluid, and restoration of MAP.

For example, the patient 120 has an estimated baseline blood volume ($_B$BV) of 5 litres, and the baseline haemoglobin concentration ($_B$Hg) prior to administration of the anaesthetic and resuscitating bolus is measured at 14 g dl$^{-1}$. Therefore, the patient 120 has an estimated total of 700 g of haemoglobin in their circulating blood volume. After administration of the resuscitating fluid and the anaesthetic, the measured haemoglobin concentration ($_M$Hg) drops to 12.73 g dl$^{-1}$. The total mass of haemoglobin within the blood circulating within the central blood compartment ($V_C$) remains unchanged during the anaesthetic and fluid administration as there is no blood loss. Therefore, the new circulating blood volume (which is then set to be the $_T$BV) is given by $_B$BV multiplied by a dilution factor ($_B$Hg/$_M$Hg, =14/12.73). Therefore, the target blood volume $_T$BV = 5 * 1.0998 = 5.5 litres.

In other embodiments of the invention, haematocrit or packed red cell volume measurements/estimates can be used to determine changes in blood volume. For example, the patient 120 has an estimated baseline blood volume ($_B$BV) of 5 litres, and the baseline haematocrit concentration prior to administration of the anaesthetic and resuscitating bolus is measured at 30%. After administration of the resuscitating fluid and the anaesthetic, the measured haematocrit drops to 25%. The total mass of haemoglobin (i.e., number of red blood cells) within the blood circulating within the central blood compartment ($V_C$) remains unchanged during the anaesthetic and fluid administration as there is no blood loss. Therefore, the new circulating blood volume (which is then set to be the $_T$BV) is given by $_B$BV multiplied by a dilution factor (30/25). Therefore, the target blood volume $_T$BV = 5 * (30/25) = 6 litres.

In some scenarios, after administration of the anaesthetic, the medical professional 122 may choose not to administer a resuscitating fluid bolus. For example, the patient's MAP may not drop below 65mmHg, and so the medical professional may consider that there is no need to administer a resuscitating fluid. In such scenarios, the target blood volume ($_T$BV) can simply be set to the baseline blood volume ($_B$BV).

Once the patient 120 is stable having received the anaesthetic, and resuscitating fluid bolus if needed, and the target blood volume ($_T$BV) has been calculated, the patient 120 can be prepared for surgery and surgery can start. As mentioned above, the circulating blood volume within the central blood compartment ($V_C$) will decrease over time due to fluid redistribution and blood loss during surgery. Using the apparatus 110 of the present invention, medical professionals 122 are able to maintain the circulating blood volume within the central compartment ($V_C$) at, or near to, the target blood volume ($_T$BV). This differs from conventional approaches wherein medical professionals administer fluids to try and maintain a mean arterial pressure without considering or defining a target blood volume to maintain.

To ensure that the circulating blood volume with the central compartment ($V_C$) is maintained near to the target blood volume ($_T$BV), the processor 112 is configured to receive ongoing monitored patient haemoglobin data. The ongoing monitored patient haemoglobin data refers to patient haemoglobin data that is obtained throughout the period for which the patient is under the influence of the anaesthetic. The processor 112 calculates a monitored blood volume ($_M$BV) based on the ongoing monitored patient haemoglobin data. The monitored blood volume ($_M$BV) refers to the real time (or substantially real time) circulating blood volume within the central compartment ($V_C$). The monitored blood volume ($_M$BV) is calculated so that the medical professional 122 can identify the volume of blood circulating in the central compartment ($V_C$), which changes over time due to the aforementioned fluid losses and the ongoing delivery of the maintenance fluid.

Using the same calculations as described above for determining the target blood volume ($_T$BV), the monitored blood volume ($_M$BV) can be calculated using the baseline blood volume ($_B$BV) and by comparing the patient's current haemoglobin concentration with the haemoglobin concentration prior to administration of the anaesthetic and resuscitating fluid bolus. The original haemoglobin concentration, determined prior to administration of the anaesthetic and resuscitating fluid bolus, is divided by the most recent value of haemoglobin concentration from the ongoing patient haemoglobin data to obtain a dilution factor, and this dilution factor is multiplied by the baseline blood volume ($_B$BV) to obtain the monitored blood volume ($_M$BV). Alternatively, the monitored blood volume can be determined by calculating the dilution factor relative to a previous monitored blood volume ($_M$BV) calculation, or by calculating the dilution factor relative to the haemoglobin concentration used to calculate the target blood volume ($_T$BV).

The monitored blood volume ($_M$BV) and target blood volume ($_T$BV) can be shown on the display 114. This enables the monitored blood volume ($_M$BV) to be compared with the target blood volume ($_T$BV) so that the medical professional 122 can determine whether more or less fluid needs to be administered to the patient 120. For example, if $_M$BV drops below $_T$BV, a fluid bolus may be given to the patient 120 intravenously or the flow rate of the maintenance fluid can be increased.

If blood loss occurs during periods of surgery, the blood loss must be taken into account when calculating $_MBV$ to account for the haemoglobin lost. To calculate $_MBV$, the processor 112 may assume that the concentration of haemoglobin in the blood lost by the patient is equal to the haemoglobin concentration of blood within the central compartment at the time of blood loss. If blood transfusions containing red blood cells are administered during surgery, the additional haemoglobin must be taken into account when calculating the $_MBV$ to account for the haemoglobin gained.

The processor 112 can receive the measured blood loss/gain and use the blood loss/gain together with the ongoing monitored patient haemoglobin data to calculate $_MBV$. For example, a medical professional 122 may measure, or estimate, the volume of blood lost by the patient 120, and this volume of blood can be input to the processor 112 via the user interface 116. As another example, a patient 120 may receive a blood transfusion of a known or estimated volume having a known or estimated haemoglobin concentration, and this volume of blood can be input to the processor 112 via the user interface 116.

The processor 112 can determine the original total haemoglobin content (e.g., Hb in grams) in the blood using the haemoglobin concentration prior to administration of the anaesthetic and fluid bolus together with the baseline blood volume ($_BBV$). The total haemoglobin lost/gained can be calculated from the volume of blood lost/gained and the haemoglobin concentration within the lost/gained blood. Therefore, the current haemoglobin content can be calculated by subtracting/adding the haemoglobin content lost/gained to the original haemoglobin content. The monitored blood volume ($_MBV$) can be calculated from the current haemoglobin concentration and the current haemoglobin content.

For example, a patient 120 with a starting haemoglobin concentration of 14 g dl$^{-1}$ and a baseline blood volume of 5L has an original total haemoglobin content of 700 g. If the patient loses 500ml of blood, which equates to 70 g (assuming the concentration of blood lost is 14 g/dl), the new total haemoglobin content is 630g. If the measured haemoglobin concentration ($_MHg$) in the ongoing patient haemoglobin data is measured to be 11.5 g dl$^{-1}$ (115 g l$^{-1}$), then $_MBV$ is calculated as 5.48L (630g divided by 115 g l$^{-1}$).

The example above assumes that all the blood lost by the patient 120 had the original haemoglobin concentration. To provide a more accurate calculation for $_MBV$, the processor 112 can continuously monitor the haemoglobin concentration across the blood loss period (this is part of the ongoing monitored patient haemoglobin data) and estimate the concentration of haemoglobin in the blood lost by averaging over the period of blood loss.

For a patient receiving a blood transfusion, similar calculations are performed, except the total haemoglobin content increases. For example, at a given point in surgery a patient 120 has a measured haemoglobin concentration of 8 g dl$^{-1}$ and the monitored blood volume ($_MBV$) is calculated as 5L, meaning the total haemoglobin content is 400g. A 250ml packed cell blood transfusion is given to the patient 120 and the transfused blood has a haemoglobin concentration of 32 g dl$^{-1}$, meaning 80 g of haemoglobin is introduced and the new total haemoglobin content is 480 g. After the transfusion is complete, if the haemoglobin concentration is measured at 9 g dl$^{-1}$, $_MBV$ is calculated to be 5.33L (480 divided by 90 g l$^{-1}$).

The processor 112 is configured to calculate a central compartment ($V_C$) fluid loss rate. The central compartment ($V_C$) fluid loss rate can represent the loss of fluid through elimination and redistribution. The processor 112 may be configured to calculate the central compartment ($V_C$) fluid loss rate using flow rate constants. The flow rate constants can represent the rate at which fluid enters or exits the central blood compartment ($V_C$).

In addition to calculating a monitored blood volume ($_MBV$) and target blood volume ($_TBV$), the processor 112 can be configured to calculate a predicted future blood volume ($_pBV$). The processor 112 can be configured to calculate the predicted future blood volume ($_pBV$) by determining the central compartment ($V_C$) fluid loss rate.

The display 114 can display the central compartment ($V_C$) fluid loss rate and/or the predicted future blood volume ($_pBV$). The display 114 can display the predicted future blood volume ($_pBV$) for a given future point in time. The display 114 can display the predicted future blood volume ($_pBV$) as a function of time.

Displaying central compartment ($V_C$) fluid loss rate enables a medical professional 122 to compare the intravenous maintenance fluid delivery rate with the central compartment ($V_C$) fluid loss rate. Displaying the predicted future blood volume ($_pBV$) enables the medical professional 122 to compare the predicted future blood volume ($_pBV$) with the target blood volume ($_TBV$). These comparisons can assist the medical professional to determine whether to increase or decrease the rate of fluid administration to the patent. For example, if the predicted future blood volume ($_pBV$) is lower than the target blood volume ($_TBV$), a medical professional may increase the flow rate of the intravenous maintenance fluid drip.

The processor 112 can provide one or more suggestions (which are displayed via display 114) based on the predicted future blood volume ($_pBV$). For example, the processor 112 may recommend increasing the maintenance fluid rate, or may recommend a fluid bolus, in response to $_pBV$ falling sufficiently far below $_TBV$. Alternatively, the processor 112 may recommend decreasing the maintenance flow rate.

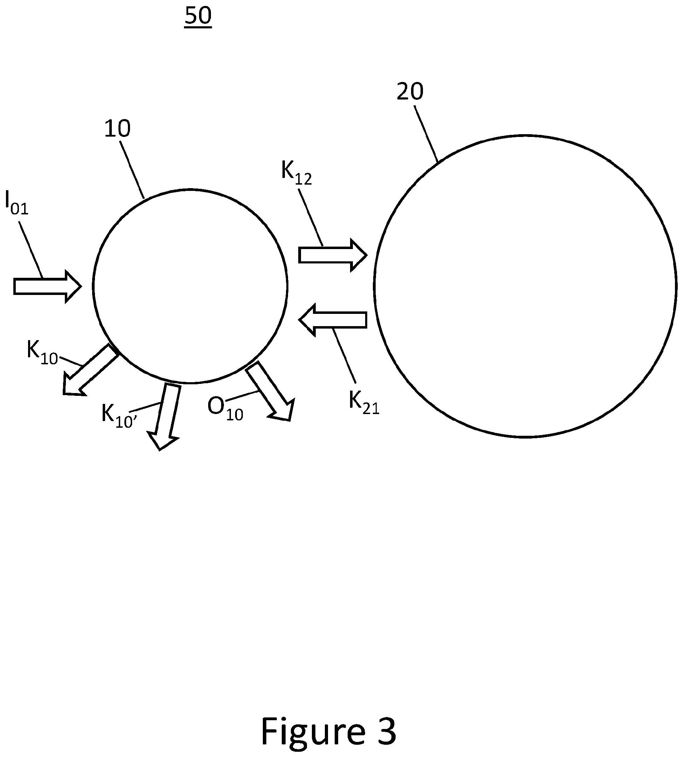

Figure 3 shows a schematic overview of a model 50 representing fluid redistribution inside the body of a patient 120. This model 50 can be used to select the relevant flow rate constants used by the processor 112 to calculate the central compartment ($V_C$) fluid loss rate and/or the predicted future blood volume ($_pBV$) according to some embodiments of the invention. In other embodiments, alternative models can be used, and alternative flow rate constants can be used by the processor 112.

The model 50 comprises two main compartments - the central blood compartment 10 and the interstitial fluid compartment 20 (also known as the "tissue space").

As explained above, the central blood compartment 10 comprises the patient's veins/venules, arteries, capillaries and heart chambers.

The interstitial fluid compartment 20 comprises fluid in the extra cellular matrix space between the capillaries and cells and bathes all cells in the body, acting as the link for micro circulation between intra cellular fluid and the fluid element (plasma) of the central blood compartment. Interstitial fluid contains/transports oxygen, glucose, amino acids, and other nutrients needed by tissue cells.

In the model 50, fluid can enter/exit the central blood compartment ($V_C$), at least, via the following pathways:

- Fluid can enter the central blood compartment 10 intravenously - via an infusion or as a bolus. This is represented in Figure 2 as the input $I_{01}$.
- Fluid can enter the central blood compartment 10 from the interstitial compartment 20.
- Fluid can exit the central blood compartment 10 by passing into the interstitial compartment 20
- Fluid can exit the central blood compartment 10, via the kidneys, via urine excretion.
- Fluid can exit the central blood compartment through insensible losses of fluid via the skin and respiratory track.
- Fluid can exit the central blood compartment 10 through blood loss which occurs during surgery. This is represented in Figure 2 as the input $O_{10}$

Therefore, the processor 112 can be configured to calculate the central compartment ($V_C$) fluid loss rate and/or the predicted blood volume ($_pBV$) using one or more of the following flow rate constants:

- $k_{10}$ - the rate at which fluid leaves $V_C$ via urine excretion;
- $k_{12}$ - the rate at which fluid leaves $V_C$ into the interstitial compartment; and
- $k_{10'}$ - the rate at which fluid leaves Vc via insensible losses;
- $k_{21}$ - the rate at which fluid enters $V_C$ from the interstitial compartment: and
- $O_{10}$ - the rate of blood loss from the Vc

In some embodiments, the flow rate constant $k_{12}$ can be used to represent the net rate of fluid flow from $V_C$ to the interstitial compartment, meaning it incorporates $k_{21}$.

In some embodiments, the flow rate constant $k_{21}$ is ignored from calculations for $_pBV$, as across the short time frame of an operation the infused fluid is assumed to be simply distributed from $V_C$ to the interstitial compartment.

In some embodiments, the flow rate constants $k_{12}$ and $k_{10}$ are functions of MAP. As MAP increases, the flow rates constants $k_{12}$ and $k_{10}$ increase.

In some embodiments of the invention, the central compartment ($V_C$) fluid loss rate and/or the predicted future blood volume ($_pBV$) can be calculated using the following differential equation:

$$\frac{dBV_C}{dt} = I_{01} - O_{10} - k_{12}(BV_C) - k_{10}(BV_C) - k_{10'}(BV_C) + constant$$

$BV_C$ represents the circulating blood volume in the central compartment ($V_C$). By solving the differential equation, $BV_C$ is determined as a function of time. $BV_C$ for a given time in the future is the predicted future blood volume ($_pBV$) which can be compared against the target blood volume ($_TBV$).

The first term in the equation ($\frac{dBV_C}{dt}$) represents the rate of change of $BV_C$.

The second term in the equation ($I_{01}$) represents the volume of fluid entering the central compartment ($V_C$) intravenously. This is given by the maintenance fluid flow rate together with any fluid bolus.

The third term in the equation represents the blood loss ($O_{10}$) during surgery.

The second and third terms may themselves be functions of time (e.g., to account for predicted blood loss and to changes to intravenous flow rates).

The fourth term in the equation ($k_{12}(BV_C)$) represents the rate of fluid flow from the central compartment ($V_C$) to the interstitial compartment.

The fifth term in the equation $k_{10}(BV_C)$ represents the rate of fluid flow out of the central compartment ($V_C$) through urinary output.

The sixth term in the equation ($k_{10'}(BV_C)$) represents the rate of fluid flow out of the central compartment ($V_C$) through insensible losses.

The processor 112 can be configured to use any known numerical method to solve the differential equation to obtain values for predicted future blood volume ($_pBV$).

To calculate the central compartment ($V_C$) fluid loss rate and/or the predicted future blood volume ($_PBV$), the processor 112 initially uses an initial set of flow rate constants. The initial set of flow rate constants can be predetermined and can be selected according to one or more user characteristics (e.g., age, height, weight, sex) and distribution/elimination covariates, such as MAP. The predetermined flow rate constants can be based on averages from historic surgery data.

In some embodiments, the processor 112 generates the initial flow rate constants in response to the input of user characteristics and distribution and elimination covariates via the user interface 116. In other embodiments, a medical professional 122 may directly input the user characteristics and distribution/elimination covariates via the user interface 116.

For example, a medical professional 122 may provide the processor 112 with values for initial estimate values for $k_{10}$, $k_{12}$, $k_{10'}$, $k_{21}$, and $O_{10}$ obtained from a look-up table taking into account patient characteristics and surgery characteristics (e.g., a given surgery may have a given predicted rate of blood loss). The medical professional may input the current infusion rate $I_{01}$ to the processor.

The processor 112 can perform initial calculation for the central compartment ($V_C$) fluid loss rate and/or the predicted future blood volume ($_PBV$) based on the initial flow rate constants.

To obtain more accurate calculations for the central compartment ($V_C$) fluid loss rate and/or the predicted future blood volume ($_PBV$), the flow rates can be updated and individualised to a specific patient. The rate of fluid redistribution/elimination for a patient 120 is unique to each patient, and so using individualised values, rather than predetermined average values, enables more accurate calculations. The flow rate constants can be updated automatically by the processor 112, manually by the medical professional, or both, throughout the period of anaesthesia so as to provide more accurate calculations.

For example, a medical professional 122 can measure total urinary output and blood loss from the patient 120 and input these values to the processor 112. The medical professional 122 can input these measurements to the processor 112 regularly. The processor 112 is configured to update the associated flow rate constants ($K_{10}$ and $O_{10}$). The flow rate constants can be updated using the measured total urinary output and blood loss from the patient 120 together with the calculated values of $_MBV$ during the period of blood loss and urine output. More specifically, if the total urine output and blood over a period of five minutes is known, and calculated values for the monitored blood volume ($_MBV$) are known for that five-minute period, the values of $k_{10}$ and $O_{10}$ can be estimated by the processor 112 using known mathematical techniques. Further, the processor 112 can update the values of the remaining flow rate constants based on the measurements for the monitored blood volume ($_MBV$) by fitting the differential equation to the $_MBV$ values using known numerical methods and mathematical techniques for curve fitting. The flow rate constants can be updated using any suitable technique, for example, using known optimisation techniques for curve fitting.

The updated flow rate constants are described as "individualised" to the patient.

By obtaining individualised flow rate constants for a patient, the rate of intravenous fluid delivery ($I_{01}$) required to maintain, or reach, $_TBV$ can be calculated by the processor 112. The display 114 can display a recommended maintenance flow rate calculated by the processor 112. If the $_MBV$ is sufficiently low compared to $_TBV$, the processor 112 can calculate a required bolus volume and rate of administration to rapidly increase $_MBV$ towards the $_TBV$. The recommendation to use a bolus by the processor 112 can be shown on the display 114.

In some embodiments, the processor 112 is configured to calculate a baseline interstitial fluid volume ($_BIFV$). The processor 112 can calculate the baseline interstitial fluid volume ($_BIFV$) using user characteristics (e.g., height, weight, age, sex). For example, the processor 112 may use the formulae described in Faucon et al 2022 "Estimating extracellular fluid volume in healthy individuals: Evaluation of existing formulae and development of a new equation". As another example, the total body water can be predicted using Watson's formula, and the extracellular portion of this water can be taken to be 44% of the total body water. The baseline interstitial fluid volume ($_BIFV$) can be taken to be the extracellular body water minus the baseline blood plasma volume, which is approximately equal to the baseline blood volume ($_BBV$) multiplied by (1 - haematocrit).

The processor 112 can be configured to calculate a monitored interstitial fluid volume ($_MIFV$). The processor 112 can be configured to calculate a predicted future interstitial fluid volume ($_PIFV$).

In a first example, the processor 112 calculates the total volume of fluid added to the interstitial compartment via the term $k_{12}(BV_C)$, or in some instances wherein $k_{21}(BV_C)$ is significant during the time of anaesthesia, the net fluid volume of fluid $k_{12}(BV_C) - k_{21}(BV_C)$, and adds this value to the baseline interstitial fluid volume ($_BIFV$) to calculate the monitored interstitial fluid volume ($_MIFV$). The predicted future interstitial fluid volume ($_PIFV$) can be calculated by monitoring the volume of fluid expected from the term $k_{12}(BV_C)$, or the net fluid volume of fluid $k_{12}(BV_C) - k_{21}(BV_C)$, and adding this value to the baseline interstitial fluid volume ($_BIFV$)

In a second example, the processor 112 calculates the total volume of fluid added to the interstitial compartment by finding the difference between the fluid and blood given to the patient and the fluid lost through blood loss, insensible losses and urination. This volume of fluid added to the interstitial compartment is then added to the initial to the baseline interstitial fluid volume ($_BIFV$) to calculate the monitored and projected interstitial fluid volume ($_MIFV$).

Monitoring the interstitial fluid volume and predicting the future the interstitial fluid volume enables medical professionals 122 to anticipate and minimise the gain in interstitial fluid volume to ensure that an excessive amount of fluid does not enter the patient's 120 interstitial fluid compartment, which has negative effects as previously described.

The processor 112 can be configured such that, if too much fluid is going to enter the interstitial fluid compartment over the predicted course of the operation, it can provide a recommendation to reduce MAP (for example, by allowing central blood volume to fall, by reducing chronotropic/inotropic drug support, or by administering a vasodilator such as nitroprusside or nitro-glycerine) to a safe but lower level, thereby reducing the Vc redistribution and elimination loss rates (i.e., in the case of the interstitial fluid this will reduce the build-up rate and allow for reduction of the maintenance fluid rate).

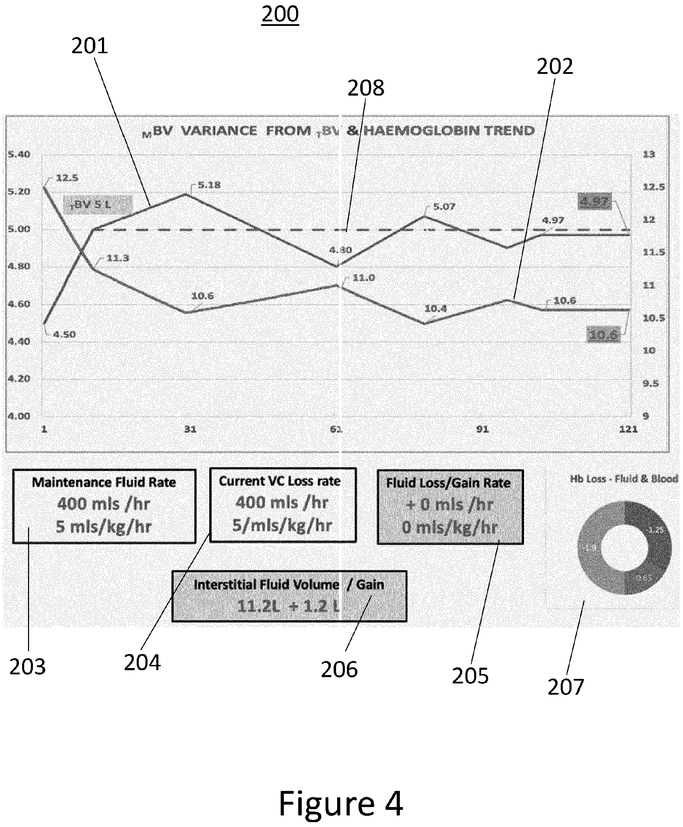

An example will now be described, with reference to Figures 4-6, to demonstrate the apparatus 110 in use.

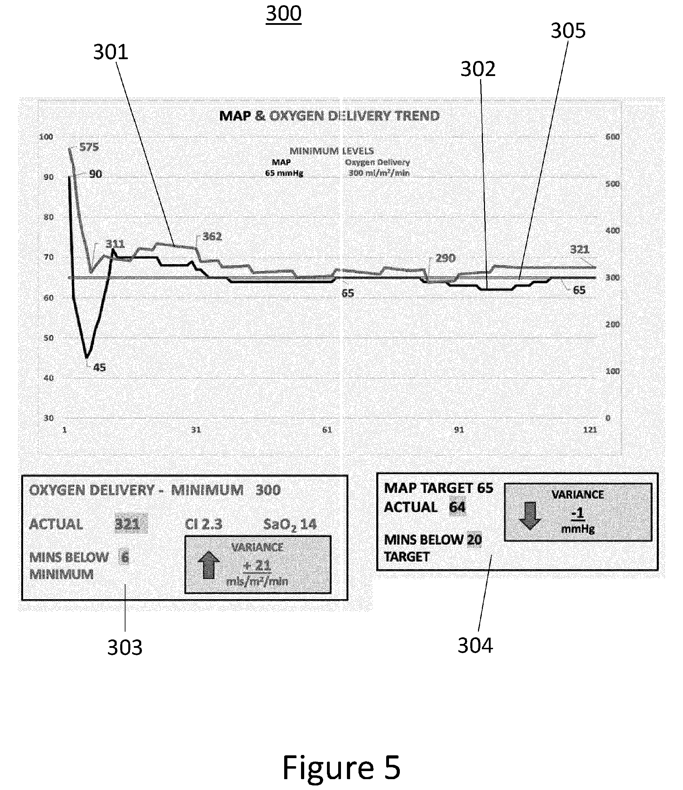

Figures 4 and 5 respectively show a blood volume screen 200 and a perfusion screen 300 which form the display 114 in some embodiments of the invention.

The blood volume and perfusion screens 200, 300 in Figures 4 and 5 show patient data for an initial period of anaesthetic induction and resuscitation followed by a period of surgery.

Figure 4 shows the blood volume screen 200. The blood volume screen 200 comprises a graph which shows the monitored blood volume ($_M$BV) against time (represented via line 201). The graph also shows the haemoglobin concentration within the blood of the central compartment ($V_C$) against time (represented via line 202). $_M$BV values are given in litres, the haemoglobin concentration values are given in grams per decilitre, and the time is given in minutes.

The blood volume screen 200 displays the current maintenance fluid rate being provided to the patient via graphic 203. The current maintenance fluid rate can be input to the processor 112, or the processor 112 can be directly connected to the device providing the maintenance fluid (e.g., a pump).

The blood volume screen 200 displays the central compartment ($V_C$) fluid loss rate via graphic 204.

The blood volume screen 200 displays the net rate of fluid gain/loss for the central blood compartment ($V_C$), which is the difference between the maintenance fluid rate and the rate of fluid loss, via graphic 205.

The blood volume screen 200 displays the monitored interstitial fluid volume ($_M$ISF) via graphic 206. The graphic 206 displays $_M$ISF as the baseline interstitial fluid volume ($_B$ISF) plus the additional fluid since the monitoring started.

The blood volume screen comprises a graphic 207 showing the total change in haemoglobin concentration ("-1.9 g dl$^{-1}$" in this example) and the contribution of blood loss and fluid dilution to the change in haemoglobin concentration. The graphic 207 comprises numbers which dynamically change during the surgery to inform the medical professional of the contribution that the fluid administered intravenously has to the haemoglobin concentration, and how this compares with direct loss of blood/haemoglobin. In the example of Figure 4, there is a decrease in the haemoglobin concentration of 0.65 g dl$^{-1}$ attributed to blood loss and a decrease in the haemoglobin concentration of 1.25 g dl$^{-1}$ attributed to dilution.

The graphics 203-207 are given as examples only. In other embodiments, alternative graphics may be used to display the same, or different information.

The data displayed in the graph which shows the monitored blood volume ($_M$BV) (line 201) and measured haemoglobin concentration within the blood of the central compartment (line 202) is described below across a period of surgery.

During the anaesthetic induction period (0-10 minutes), the anaesthetic is administered, causing vasodilation in the patient 120. As described above, the anaesthetist administers an intravenous fluid bolus (i.e., the resuscitating fluid) to the patient to restore MAP to a safe level following administration of the anaesthetic. The addition of fluid to the central compartment ($V_C$) increases the circulating blood volume, which causes the $_M$BV value calculated by the processor to increase. The addition of the fluid also dilutes the concentration of haemoglobin, causing the measured haemoglobin concentration ($_M$Hb) to decrease. The haemoglobin concentration ($_M$Hb) falls from 12.5 to 11.3 g dl$^{-1}$.

At the end of the initial ten-minute period, the patient is haemodynamically stable (i.e., MAP and cardiac output are at a safe level). The monitored blood volume $_M$BV circulating in the central compartment ($V_C$) is calculated to be 5L. Therefore, the target blood volume $_T$BV is set at 5L. The target blood volume ($_T$BV) is displayed via line 208.

After the initial resuscitation fluid bolus is given to the patient 120, a maintenance fluid is given to the patient 120 by the medical professional 122. The maintenance fluid is given intravenously at a constant rate of 6 mls /kg /hr. The maintenance fluid is given continuously from 10 minutes onwards. The maintenance fluid is given to account for fluid loss and to try and maintain $_M$BV near to $_T$BV.

During the period from 10-30 minutes, there is blood loss of 300ml caused by the surgery. To compensate for this blood loss and to compensate for other fluid losses from the central compartment ($V_C$), a fluid bolus of 250ml is given to the patient 120, in addition to the maintenance fluid.

The combined effect of dilution (from both the maintenance fluid and the fluid bolus) together with the loss of haemoglobin through blood loss causes the haemoglobin concentration to fall from 11.3 to 10.6 g dl$^{-1}$. The medical professional 122 measures the blood loss and inputs the measured volume to the processor 112. As described earlier, the processor 112 takes the loss of haemoglobin into account when calculating $_M$BV. The monitored blood volume ($_M$BV) increases from 5L to 5.18L between 10-30 minutes.

From 30 minutes to 60 minutes, the patient 120 still receives the maintenance fluid but is not given a fluid bolus. As such, the total amount of fluid being given to the patient intravenously is lower between 30-60 minutes than it is between 10 to 30 minutes. The rate of fluid loss from the central compartment ($V_C$) is greater than the rate at which the maintenance fluid is provided to the patient, meaning the circulating blood volume in the central compartment ($V_C$) decreases and the calculated values for $_MBV$ decrease from 5.18L to 4.8L. The measured haemoglobin concentration values increase from 10.6 to 11 g dl$^{-1}$.

From 60-80 minutes, the rate at which the maintenance fluid is given to the patient 120 is increased by the medical professional 122. The increase means that maintenance fluid is added into the central compartment at a greater rate than fluid is lost from the central compartment. This leads to a decrease in measured haemoglobin concentration from 11 to 10.4 g dl$^{-1}$ and an increase in calculated $_MBV$ from 4.8L to 5.07L.

From 80-95 minutes, the maintenance flow rate is reduced to prevent $_MBV$ from going too far above $_TBV$, which would lead to excess levels of fluid entering the interstitial fluid compartment. The reduced maintenance flow rate is less than the rate of fluid loss from $V_C$, so the measured haemoglobin concentration increases and the calculated $_MBV$ falls.

From 95-100 minutes, the maintenance flow rate is increased because $_MBV$ is below $_TBV$. The maintenance flow rate exceeds the rate of fluid loss from $V_C$ so the measured haemoglobin concentration decreases and the calculated $_MBV$ increases.

From 100 minutes onwards, the maintenance fluid rate is adjusted to match the rate of fluid loss rate. Therefore, the measured haemoglobin concentration and calculated $_MBV$ remain constant. The medical professional 122 matches maintenance fluid supply rate to the central compartment loss rate at this stage because $_MBV$ is approximately equal to $_TBV$.

Throughout the period of time shown in the graph, the medical professional 122 measures the urinary output from the patient and inputs this data to the processor 112.

During the period of time shown above, the processor 112 continuously updates the estimated fluid loss rate from the central compartment. The processor starts with an initial fluid loss rate, and adjusts this value based on the change in $_MBV$ over time. The fluid loss rate is displayed via graphic 204. The longer the processor has to estimate the fluid loss rate, the more accurate the estimate becomes. This is why the medical professional 122 is able to bring the maintenance fluid rate closer to, and eventually match, the fluid loss rate.

The perfusion screen 300 is displayed throughout the surgery together with the blood volume screen 200. The corresponding data for the same patient during the same time period is described below in relation to the perfusion screen 300 with reference to Figure 5.

Figure 5 shows the perfusion screen 300. The perfusion screen 300 comprises a graph which shows the oxygen delivery rate (bound oxygen measured in ml m$^{-2}$ min$^{-1}$) against time (represented via line 301). The graph also shows the MAP (measured in mm Hg) against time (represented via line 302).

The perfusion screen 300 displays a graphic 303. The graphic 303 displays the current oxygen delivery rate (i.e., the current value from line 301) together with a minimum oxygen delivery rate for which there is sufficient organ perfusion. The oxygen delivery rate is calculated by monitoring various physiological parameters (i.e., cardiac index and arterial oxygen content), as described earlier. The minimum oxygen delivery rate can be set by the medical professional 122. In this example, the minimum oxygen delivery rate is 300 ml m$^{-2}$ min$^{-1}$. The graphic 303 shows the difference between the current and the minimum oxygen delivery rate.

The graphic 303 shows the total amount of time for which the patient's oxygen delivery rate has been below the minimum oxygen delivery rate.

The graphic 303 shows measured physiological parameters. In this example, the graphic 303 shows cardiac index (cardiac output / body surface area) levels and arterial oxygen content levels.

The perfusion screen 300 displays a graphic 304. The graphic 304 displays the measured arterial pressure, in this case MAP, together with a minimum MAP. The minimum MAP is set by the medical professional 122 and is the minimum MAP which the medical professional 122 considers to be safe. The graphic 304 also displays the total amount of time for which the measured MAP has been below the minimum MAP.

During the anaesthetic induction period, the anaesthetic is administered, causing vasodilation. This leads to a drop in blood flow which causes a drop in MAP from 90 to 45 and a decrease in the oxygen delivery rate from 575 to 311. The anaesthetist then administers a resuscitating fluid bolus to increase the patient's MAP back above the target MAP but below the pre induction MAP of 90. The oxygen delivery rate increases with MAP, but the oxygen delivery rate does not increase as significantly due to the dilution of the haemoglobin concentration and reduced blood flow.

Throughout the surgery period, the MAP remains stable around the target MAP, but the oxygen delivery rate remains low and drops repeatedly below the minimum oxygen delivery rate. The oxygen delivery rate remains low, despite the safe level of MAP, because of the low haemoglobin concentration caused by the dilution and blood loss and the lower level of blood flow post anesthesia induction.

At 120 minutes, the oxygen delivery rate is low, and the patient is building up a significant oxygen debt and associated lactic acid build-up. Therefore, the medical professional 122 wants to increase it to a higher level (e.g., of 350 mls/m$^2$/min, which is an increase of i.e. by + 17%). There are a number of options open to the medical professional that may increase blood flow in order to improve the oxygen delivery. Without the present invention, if the medical professional 122 only had access to MAP measurements, and did not have the blood volume screen 200, they may think that fluid losses have been higher than the maintenance flow rate and that they need to increase the blood volume (e.g., through a fluid bolus). However, the use of the blood volume screen 200 enables the medical professional to realise that: (a) the blood volume is already at the target blood volume; and (b) the introduction of additional fluid beyond the present maintenance flow rate would lead to a further decrease in haemoglobin concentration.

In this scenario, administration of additional fluid will likely only increase blood flow in 50% of cases at this stage of the operation. Additionally, with a monitored blood volume ($_MBV$) of 5 litres you can expect a fall in arterial oxygen content of -5% for every 250mls increment of the circulating blood volume. Clearly, there are considerable risks with giving extra fluid: there may be no improvement in blood flow; and a drop in oxygen content, and hence a reduction in oxygen delivery in response to fluid, cannot be excluded. Even in the 50% of fluid responders, achieving a 17% increase in oxygen delivery rate is very challenging, particularly in elderly patients, as the cardiac index would actually have to be increased by 22% from 2.3 to 2.8 l/min/m$^2$ in order to both offset the haemodilution of 5% and improve blood flow by 17%. Therefore, consideration of the information in the displays 200, 300 makes it clear to the medical professional 122 that alternative methods to increase the oxygen delivery rate should be considered, these could include an infusion of chronotropic / inotropic drugs (e.g., dobutamine/dopamine/noradrenaline/ milrinone) which increase the force and/or rate of the heartbeat, thereby increasing blood flow and hence oxygen delivery. In other cases, where the haemoglobin concentration is lower, a blood transfusion may be the best option this would increase the arterial oxygen content of the blood and reduce the extent that the blood flow would need to be increased to achieve the desired oxygen delivery rate.

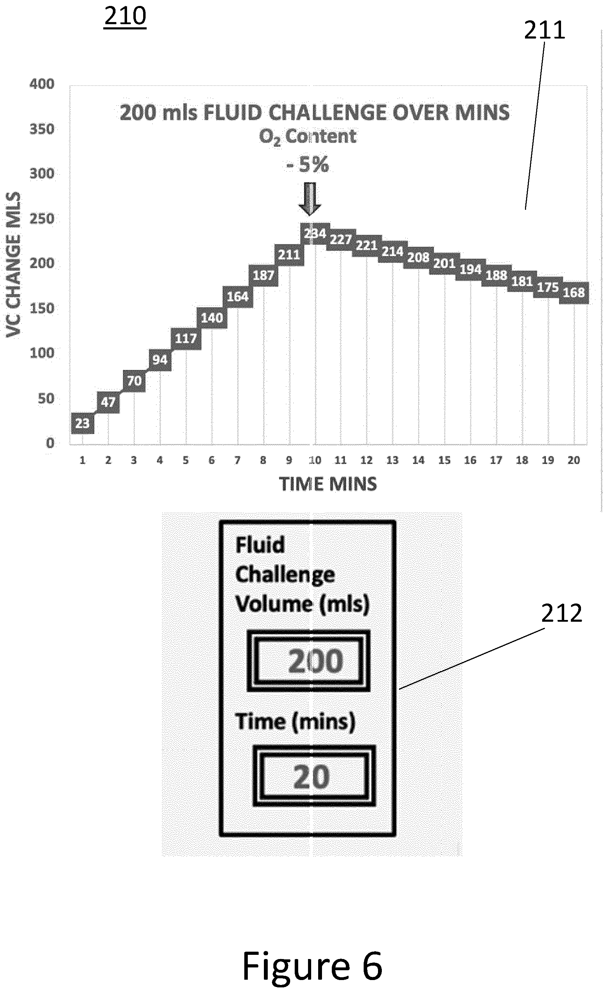

The blood volume screen 200 can also include a fluid challenge calculator 210, as shown in Figure 6. The fluid challenger calculator 210 can be used by a medical professional to assess and interpret the effects that providing a fluid challenge (i.e., a fluid bolus) would have on the patient's 120 blood volume.

The fluid challenger calculator 210 comprises a graph 211 and an input graphic 212. The input graphic 212 displays the volume of the fluid bolus together with the time the fluid delivery is going to be spread over. The graph 211 shows how the circulating blood volume within the central compartment will change over that time period. The data on the graph 211 is calculated by the processor 112 using the calculated fluid loss rate from the central compartment. The graph 211 may instead display the predicted future blood volume ($_pBV$) against time for a fluid challenge.

For the patient 120 of Figures 4 and 5, at 120 minutes, the medical professional 122 may use the fluid challenge calculator 210 to assess how effective a fluid bolus might be, as it would increase MAP by increasing cardiac output.

If the circulating blood volume is increased from 5000 to 5234 ml, the haemoglobin concentration will fall by approximately 5%. The fall in haemoglobin concentration can be displayed on the graph 211. So, for the fluid bolus to increase the cardiac output by 17% net, then it would have to increase the cardiac output by 22% (to offset the dilution of 5% & increase by net 17%). 22% is a large increase to expect, given that the blood volume is already enlarged from 4.5 to 5L. This would further demonstrate to the medical professional 122 that alternative methods for increasing oxygen delivery should be considered.

In some embodiments of the invention, there is a system for training management of fluid administration to a patient following administration of an anesthetic or in other medical procedures.

The system for training may be configured to simulate patient response to fluid resuscitation and maintenance fluid, illustrating the changes in central volume and interstitial volume during the course of a surgical procedure or other medical procedure. The system may comprise a user interface with control elements for the user to define a target blood volume, arterial blood pressure and oxygen delivery targets and virtually administer: a resuscitation fluid and any fluid bolus and/or maintenance flow, blood products, various cardiac and vasoactive drugs. The user interface may comprise one or more control elements (e.g., buttons, sliders, dialog boxes) for inputs such as: maintenance fluid flow rates, fluid bolus injections (volume and rate), resuscitation volumes (volume and rate), one or more drugs, or blood transfusions. The user interface may comprise any of the graphics and features described with reference to Figures 4, 5 and 6, which may assist the user to determine an appropriate fluid management strategy.

In some scenarios, the system may withhold some parameters from being displayed, such as the target blood volume - in order to emphasize the value of these parameters in appropriate fluid management. In some scenarios, the system may rerun a simulation without and without certain parameters in order to emphasize the importance of these parameters.

The simulation may be based on a model or determined by analysis of real patient data from a surgical procedure. For example, the fluid loss terms discussed above (with reference to the model) may be determined for a real procedure, and a user may be tested using the realistic data derived from the scenario to administer fluid appropriately. A scoring metric may be determined from the simulation, which may be based on one or more of: maintenance of a suitable MAP (positive score), suitable oxygen delivery without excessive hemodilution and requirement for excessive inotrope support (positive score), excess interstitial fluid (negative score). The simulation may be based on randomly generated data (i.e., randomly generated physiological parameters, randomly generated flow rate constants, randomly generated losses, etc.). The randomly generated data may be generated based on real historical data so as to ensure the simulation is realistic.

The system for training may comprise the display described in Figures 4-6 with the blood volume and perfusion screens.

The simulation may also facilitate retrospective analysis of real scenarios - to illustrate whether performance in a real setting was optimal or not. Analysis of such historical information may provide an invaluable training aid.

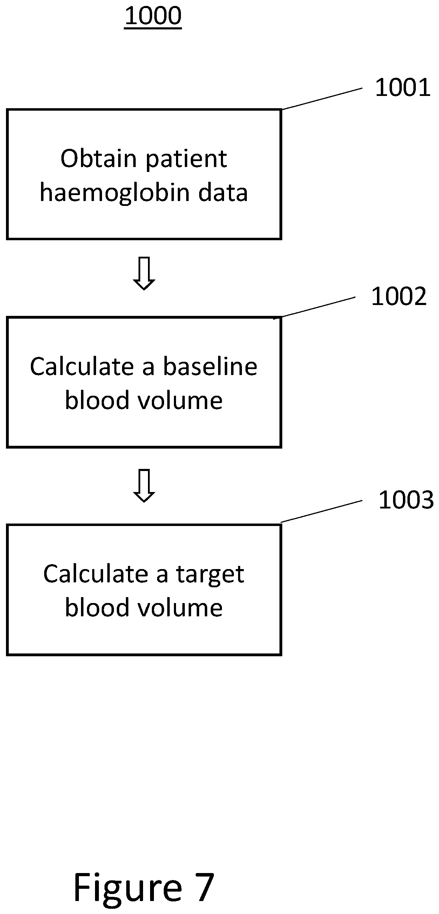

Figure 7 shows a flowchart 1000 representing a method for managing fluid administration to a patient following administration of an anaesthetic.

The method comprises obtaining 1001 patient haemoglobin data before and after the administration of the anaesthetic and a resuscitating fluid to the patient.

The method comprises calculating 1002 a baseline blood volume ($_B$BV) of the patient.

The method comprises, after administration of the anaesthetic and the resuscitating fluid, calculating 1003 a target blood volume ($_T$BV), wherein the target blood volume ($_T$BV) is calculated by comparing the monitored patient haemoglobin data before and after the administration of the anaesthetic and resuscitating fluid.

The method can include any steps performed by the processor 112, medical professional 122, and/or the patient monitoring device 118.

Throughout this specification reference is made the mean arterial pressure (MAP). The skilled person will recognise that other measures of arterial pressure, such as systolic arterial pressure, can be used instead of MAP in various embodiments of the invention.

**Claims**

1. A system for managing fluid administration to a patient following administration of an anaesthetic, comprising:
   a processor configured to:

   receive monitored patient haemoglobin data before and after the administration of the anaesthetic to the patient and, if administered, an initial resuscitating fluid bolus, wherein patient haemoglobin data comprises one or more of: haemoglobin concentration, haematocrit, or packed red cell volume;
   calculate a baseline blood volume ($_B$BV) of the patient;
   after administration of the anaesthetic and, if administered, an initial resuscitating fluid bolus, calculate the patient blood volume by comparing the monitored patient haemoglobin data before and after the administration of the anaesthetic and, if administered, the initial resuscitating fluid bolus;
   set the calculated patient blood volume as a target blood volume ($_T$BV);
   receive ongoing monitored patient haemoglobin data; and
   calculate a monitored blood volume ($_M$BV) based on the ongoing monitored patient haemoglobin data;
   calculate a predicted blood volume; and
   calculate, based on the predicted blood volume ($_P$BV) and the target blood volume ($_T$BV), one or more of:

      (i) a recommended maintenance fluid rate to maintain $_M$BV at, or near to, $_T$BV; and
      (ii) a recommended resuscitative fluid bolus volume and rate of administration to bring $_M$BV to, or near to, $_T$BV.

2. The system of claim 1, wherein the processor is further configured to:

   receive user characteristics; and
   calculate the baseline blood volume ($_B$BV) using the user characteristics.

3. The system of claim 1 or claim 2, wherein the processor is configured to calculate the target blood volume ($_T$BV) by multiplying the baseline blood volume ($_B$BV) by a dilution factor.

4. The system of any preceding claim, wherein the processor is further configured to:

   receive a net change in haemoglobin content; and
   calculate the monitored blood volume ($_M$BV) based on the ongoing monitored patient haemoglobin data together with the net change in haemoglobin content.

5. The system of any preceding claim, wherein the processor is configured to calculate a central compartment fluid loss rate.

6. The system of claim 5, wherein the processor is configured to update the central compartment fluid loss rate such that it is individualized to the patient.

7.  The system of claim 5 or claim 6, wherein the processor is configured to calculate the central compartment fluid loss rate using a plurality of flow rate constants.

8.  The system of claim 7, wherein the processor is configured to update the flow rate constants such that they are individualized to the patient.

9.  The system of claim 8, wherein the processor is configured to:

    receive at least one of: measured urinary output, measured blood loss or blood gain, and patient MAP; and update the flow rate constants such that they are individualized to the patient based on the measured urinary output, measured blood loss or blood gain, and/or patient MAP.

10. The system of any of claims 7-9, wherein the flow rate constants comprise one or more of:

    (i) rate of fluid loss to the urinary system of the patient;
    (ii) rate of fluid loss to the interstitial fluid compartment of the patient;
    (iii) rate of insensible fluid losses; and
    (iv) rate of blood loss

11. The system of any preceding claim, wherein the processor is configured to:

    estimate an initial interstitial fluid volume; and
    calculate a monitored interstitial fluid volume ($_M$ISF).

12. The system of any preceding claim, wherein the system comprises a display, wherein the display is configured to show:

    (i) target blood volume ($_T$BV) and monitored blood volume ($_M$BV); and/or
    (ii) the difference between the target blood volume ($_T$BV) and the monitored blood volume ($_M$BV).

**Patentansprüche**

1.  System zum Verwalten einer Flüssigkeitsverabreichung an einen Patienten, folgend auf eine Verabreichung eines Betäubungsmittels, umfassend:
    einen Prozessor, der zu Folgendem konfiguriert ist:

    Empfangen überwachter Patientenhämoglobindaten vor und nach der Verabreichung des Betäubungsmittels an den Patienten und, falls verabreicht, eines anfänglichen Wiederbelebungsflüssigkeitsbolus, wobei die Patientenhämoglobindaten eines oder mehrere von Folgendem umfassen: einer Hämoglobinkonzentration, eines Hämatokrits oder eines gepackten Volumens roter Blutkörperchen;
    Berechnen eines Grundblutvolumens ($_B$BV) des Patienten;
    nach der Verabreichung des Betäubungsmittels und, falls verabreicht, eines anfänglichen Wiederbelebungsflüssigkeitsbolus, Berechnen des Patientenblutvolumens durch Vergleichen der überwachten Patientenhämoglobindaten vor und nach der Verabreichung des Betäubungsmittels und, falls verabreicht, des anfänglichen Wiederbelebungsflüssigkeitsbolus;
    Einstellen des berechneten Patientenblutvolumens als ein Zielblutvolumen ($_T$BV);
    Empfangen laufender überwachter Patientenhämoglobindaten; und
    Berechnen eines überwachten Blutvolumens ($_M$BV) basierend auf den laufenden überwachten Patientenhämoglobindaten;
    Berechnen eines vorhergesagten Blutvolumens; und
    Berechnen, basierend auf dem vorhergesagten Blutvolumen (pBV) und dem Zielblutvolumen ($_T$BV), eines oder mehrerer von Folgendem:

        (i) einer empfohlenen Flüssigkeitsrate, um das $_M$BV auf oder nahe dem $_T$BV zu halten; und
        (ii) einem empfohlenen Wiederbelebungsflüssigkeitsbolusvolumen und einer empfohlenen Verabreichungsrate, um das $_M$BV auf oder nahe dem $_T$BV zu bringen.

2. System nach Anspruch 1, wobei der Prozessor ferner zu Folgendem konfiguriert ist:

    Empfangen von Benutzereigenschaften; und
    Berechnen des Grundblutvolumens ($_B$BV) unter Verwendung der Benutzereigenschaften.

3. System nach Anspruch 1 oder Anspruch 2, wobei der Prozessor dazu konfiguriert ist, das Zielblutvolumen ($_T$BV) durch Multiplizieren des Grundblutvolumens ($_B$BV) mit einem Verdünnungsfaktor zu berechnen.

4. System nach einem der vorhergehenden Ansprüche, wobei der Prozessor ferner zu Folgendem konfiguriert ist:

    Empfangen einer Nettoänderung eines Hämoglobingehalts; und
    Berechnen des überwachten Blutvolumens ($_M$BV) basierend auf den laufenden überwachten Patientenhämoglobindaten, zusammen mit der Nettoänderung des Hämoglobingehalts.

5. System nach einem der vorhergehenden Ansprüche, wobei der Prozessor dazu konfiguriert ist, eine zentrale Kompartimentsflüssigkeitsverlustrate zu berechnen.

6. System nach Anspruch 5, wobei der Prozessor dazu konfiguriert ist, die zentrale Kompartimentsflüssigkeitsverlustrate derart zu aktualisieren, dass sie für den Patienten individualisiert ist.

7. System nach Anspruch 5 oder Anspruch 6, wobei der Prozessor dazu konfiguriert ist, die zentrale Kompartimentsflüssigkeitsverlustrate unter Verwendung einer Vielzahl von Fließratenkonstanten zu berechnen.

8. System nach Anspruch 7, wobei der Prozessor dazu konfiguriert ist, die Fließratenkonstanten derart zu aktualisieren, dass sie für den Patienten individualisiert sind.

9. System nach Anspruch 8, wobei der Prozessor zu Folgendem konfiguriert ist:

    Empfangen mindestens eines von Folgendem: einer gemessenen Harnausgabe, einem gemessenen Blutverlust oder Blutgewinn und eines MAD des Patienten; und
    derartigem Aktualisieren der Fließratenkonstanten, dass sie für den Patienten individualisiert sind, basierend auf der gemessenen Harnausgabe, dem gemessenen Blutverlust oder Blutgewinn und/oder dem MAD des Patienten.

10. System nach einem der Ansprüche 7-9, wobei die Fließratenkonstanten eine oder mehrere von Folgendem umfassen:

    (i) einer Flüssigkeitsverlustrate an das Harnsystem des Patienten;
    (ii) einer Flüssigkeitsverlustrate an das Interstitialflüssigkeitskompartiment des Patienten;
    (iii) einer Rate nicht erfassbarer Flüssigkeitsverluste; und
    (iv) einer Blutverlustrate.

11. System nach einem der vorhergehenden Ansprüche, wobei der Prozessor zu Folgendem konfiguriert ist:

    Schätzen eines anfänglichen Interstitialflüssigkeitsvolumens; und
    Berechnen eines überwachten Interstitialflüssigkeitsvolumens ($_M$ISF).

12. System nach einem der vorhergehenden Ansprüche, wobei das System eine Anzeige umfasst, wobei die Anzeige dazu konfiguriert ist, Folgendes zu zeigen:

    (i) das Zielblutvolumen ($_T$BV) und das überwachte Blutvolumen ($_M$BV); und/oder
    (ii) den Unterschied zwischen dem Zielblutvolumen ($_T$BV) und dem überwachten Blutvolumen ($_M$BV).

**Revendications**

1. Système de gestion de l'administration de fluides à un patient après l'administration d'un anesthésique, comprenant : un processeur configuré pour :

   recevoir des données d'hémoglobine du patient surveillées avant et après l'administration de l'anesthésique au patient et, s'il a été administré, d'un bolus initial de liquide de réanimation, les données d'hémoglobine du patient comprenant un ou plusieurs éléments parmi : la concentration d'hémoglobine, l'hématocrite ou le volume de globules rouges concentrés ;
   calculer le volume sanguin de base ($_B$BV) du patient ;
   après l'administration de l'anesthésique et, s'il a été administré, d'un bolus initial de liquide de réanimation, calculer le volume sanguin du patient en comparant les données d'hémoglobine du patient surveillées avant et après l'administration de l'anesthésique et, s'il a été administré, du bolus initial de liquide de réanimation ;
   définir le volume sanguin calculé du patient comme volume sanguin cible ($_T$BV) ;
   recevoir des données d'hémoglobine du patient surveillées en continu ; et
   calculer un volume sanguin surveillé ($_M$BV) en fonction des données d'hémoglobine du patient surveillées en continu ;
   calculer un volume sanguin prédit ; et
   calculer, en fonction du volume sanguin prédit ($_P$BV) et du volume sanguin cible ($_T$BV), un ou plusieurs des éléments suivants :

   (i) un taux de liquide d'entretien recommandé pour maintenir $_M$BV à, ou proche de, $_T$BV ; et
   (ii) un volume de bolus de liquide de réanimation recommandé et un débit d'administration pour amener $_M$BV à, ou proche de, $_T$BV.

2. Système selon la revendication 1, dans lequel le processeur est en outre configuré pour :

   recevoir des caractéristiques d'utilisateur ; et
   calculer le volume sanguin de base ($_B$BV) à l'aide des caractéristiques d'utilisateur.

3. Système selon la revendication 1 ou la revendication 2, dans lequel le processeur est configuré pour calculer le volume sanguin cible ($_T$BV) en multipliant le volume sanguin de base ($_B$BV) par un facteur de dilution.

4. Système selon l'une quelconque des revendications précédentes, dans lequel le processeur est en outre configuré pour :

   recevoir un changement net dans la teneur en hémoglobine ; et
   calculer le volume sanguin surveillé ($_M$BV) en fonction des données d'hémoglobine du patient surveillées en continu ainsi que du changement net de la teneur en hémoglobine.

5. Système selon l'une quelconque des revendications précédentes, dans lequel le processeur est configuré pour calculer un taux de perte de fluide du compartiment central.

6. Système selon la revendication 5, dans lequel le processeur est configuré pour mettre à jour le taux de perte de liquide du compartiment central de telle sorte qu'il soit individualisé en fonction du patient.

7. Système selon la revendication 5 ou la revendication 6, dans lequel le processeur est configuré pour calculer le taux de perte de fluide du compartiment central à l'aide d'une pluralité de constantes de débit.

8. Système selon la revendication 7, dans lequel le processeur est configuré pour mettre à jour les constantes de débit de sorte qu'elles soient individualisées en fonction du patient.

9. Système selon la revendication 8, dans lequel le processeur est configuré pour :

   recevoir au moins l'un des éléments suivants : le débit urinaire mesuré, la perte ou le gain sanguin mesuré et MAP du patient ; et
   mettre à jour les constantes de débit de sorte qu'elles soient individualisées en fonction du patient en fonction du débit urinaire mesuré, de la perte ou du gain sanguin mesuré et/ou de MAP du patient.

**10.** Système selon l'une quelconque des revendications 7 à 9, dans lequel les constantes de débit comprennent une ou plusieurs de éléments suivants :

(i) un taux de perte de liquide dans le système urinaire du patient ;
(ii) un taux de perte de liquide dans le compartiment du liquide interstitiel du patient ;
(iii) un taux de pertes de fluides insensibles; et
(iv) un taux de perte de sang

**11.** Système selon l'une quelconque des revendications précédentes, dans lequel le processeur est configuré pour :

estimer un volume initial de fluide interstitiel ; et
calculer un volume de liquide interstitiel surveillé ($_M$ISF).

**12.** Système selon l'une quelconque des revendications précédentes, dans lequel le système comprend un affichage, l'affichage étant configuré pour afficher :

(i) Un volume sanguin cible ($_T$BV) et un volume sanguin surveillé ($_M$BV) ; et/ou
(ii) la différence entre le volume sanguin cible ($_T$BV) et le volume sanguin surveillé ($_M$BV).

100

110

114

112

116

Figure 1

Figure 2

$50$

$20$

$10$

$K_{12}$

$I_{01}$

$K_{10}$

$K_{10'}$

$O_{10}$

$K_{21}$

Figure 3

Figure 4

300

301

305

302

**MAP & OXYGEN DELIVERY TREND**

MINIMUM LEVELS
MAP                    Oxygen Delivery
65 mmHg                300 ml/m²/min

575

90

311

362

290

321

65

65

45

OXYGEN DELIVERY - MINIMUM   300

ACTUAL        321          CI 2.3        SaO₂ 14

MINS BELOW   6                    VARIANCE
MINIMUM                           + 21
                                  mls/m²/min

303

MAP TARGET 65                    VARIANCE
ACTUAL      64                      -1
                                   mmHg
MINS BELOW 20
TARGET

304

# Figure 5

Figure 6

1000

Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010081942 A1


**Non-patent literature cited in the description**

- **FAUCON et al.** Estimating extracellular fluid volume in healthy individuals. *Evaluation of existing formulae and development of a new equation*, 2022